# EUROPEAN PATENT APPLICATION

(11) **EP 0 953 432 A1**
(43) Date of publication of application: **03.11.1999**
(21) Application number: 98201378.1
(22) Date of filing: 28.04.1998
(51) Int. Cl.: B29C 65/54, B29C 65/42, C09J 5/10, B27G 11/00, A61B 17/00

(54) **Method and device for interconnecting two objects**

(71) Applicant: ACADEMISCH ZIEKENHUIS UTRECHT, NL-3508 GA Utrecht (NL); Rijksuniversiteit Utrecht, 3584 CS Utrecht (NL)
(72) Inventor: Borst, Cornelius, 3723 KB Bilthoven (NL)
(74) Representative: de Bruijn, Leendert C.

(57) **Abstract**

The invention relates to a method and device of for interconnecting two objects, in particular for bonding of tissue layers. The method comprises the steps of:
- placing the objects in a contacting relationship along an interface,
- penetrating the objects with a hollow delivery tube having an outlet opening and forming a channel extending on each side of the interface, transversely hereto, and
- retracting the delivery tube from the channel while supplying a bonding material via the outlet opening such that the channel on both sides of the interface is at least partly filled with the bonding material.

The method provides controlled bonding in a relatively simple and reliable manner, in particular at positions which can be accessed only with difficulty. The method and device according to the present invention are particularly suitable in combination with endoscopic surgery.

## Description

### Background of the invention

The invention relates to a method and device for interconnecting two objects, in particular a tissue bonding method and tissue bonding device.

In US patent nr. 5,209,776 a large number of methods for bonding separated tissues and vessels is described. Mechanical bonding methods comprise suturing with needle and suture wire or stapling. An alternative to suturing or stapling is adhesive bonding, using adhesives containing a concentrate of fibrinogen and thrombin or non-biological materials such as isobutyl-2-cyanoacrylate. Other, experimental, tissue bonding methods rely on local heat application to the apposed tissue layers by means of laser irradiation. The laser tissue welding bond initially is not very strong. Laser activated adhesives may be used to form a weld which bonds separated tissues together. In spite of all the above methods, suturing in general practise is the predominant tissue bonding method.

Suturing with a curved needle requires, in addition to manual dexterity, a very complicated manual manoeuvre in which the two tissue layers are penetrated by the curved needle which is returned to the point where a knot can be tied. Up to ten knots on top of each other may be required to avoid slipping of the knot. Also the tightening of the knot itself is quite complicated such that bonding by suturing requires sufficient available space and time.

In the past decade, minimally invasive, i.e. video-assisted, port hole, endoscopic surgery has replaced a number of open access surgical procedures in the abdomen and in the chest. Conventional suturing, however, is demanding through endoscopic access. Endoscopic surgery would greatly benefit from a facilitated method of tissue bonding, in addition to stapling. In particular, endoscopic vascular anastomosis surgery would benefit, because it is especially demanding if the vessels are small, as for example in coronary bypass surgery (Minimally Invasive Coronary Artery Bypass Grafting, MICABG). Open access surgery, however, may also benefit from a facilitated tissue bonding method.

### Objects of the invention

It is therefore an object of the present invention to provide a bonding method and apparatus, in particular a tissue bonding method and apparatus in which quick and reliable bonding can be achieved.

It is a further object of the present invention to provide a bonding method and apparatus in which bonding, in particular of tissue layers, can be achieved under conditions of difficult access to the layers.

It is again a further object of the present invention to provide a bonding method and apparatus in which the bond geometry and bond strength can be easily varied according to local demands.

It is finally an object of the present invention to provide a bonding method and apparatus which can be easily operated using a minimum of manual manoeuvres of reduced complexity.

### Summary of the invention

Thereto the method according to the present invention comprises the steps of :
- placing the objects in a contacting relationship along an interface,
- penetrating the objects with a hollow delivery tube having an outlet opening, and forming a channel extending on each side of the interface, transversely thereto, and
- retracting the delivery tube from the channel while supplying a bonding material via the outlet opening such that the channel on both sides of the interface is at least partly filled with the bonding material.

Contrary to the known adhesive bonding methods, the bonding material according to the present invention is applied in a direction transversely to the interface instead of parallel thereto. Thereby the materials can first be apposed in their proper position, similar to suturing, and thereafter be bonded. Contrary to known adhesive bonding methods wherein the adhesive is first applied and the tissue layers are thereafter apposed in their proper position there is no risk of contaminating the bond area with adhesive at undesired positions.

In contrast with the conventional suturing methods, the adhesive bonding method of the present invention recognises that the bond strength is achieved from the interaction of the bonding material with the sidewalls of the channel that is formed in the layers to be bonded (e.g. tissue layers). Thereby it is not necessary to lead the bonding material along a curved trajectory which crosses the interface plane twice for attaching the ends of the bonding material, as is the case in conventional suturing procedures.

Using the method according to the present invention, the bond strength can be varied by the thickness of the bonding material, which can be controlled by the retraction speed of the delivery tube and the rate of supply of bonding material via the outlet opening. Furthermore, the bond strength can be varied to match local circumstances and materials of the objects to be bonded simply by changing the composition of the bonding material, the number of bond sites per unit area and/or the orientation or configuration of the bonds.

Preferably the bonding material is an adhesive bonding material. Extrusion of the adhesive from the delivery tube forms glue wires that attach the apposed layers. Since the surface of the adhesive in the bond areas that are formed according to the present invention is large, each glue wire will provide a high individual bond strength. The overall bonding of the layers depends on the individual bond strength of each glue wire and on the number of glue wires per unit area. In addition, polymerisation of the glue can be relatively fast in case the glue activation is based on tissue contact, or water activation. Furthermore, a large surface to volume ratio prevents excessive local heat dissipation owing to polymerisation and thus prevents thermal tissue damage.

The bonding material is preferably formed by a curable, flowable material, such as an adhesive curing upon the contact with water or air. However, the bonding material may also comprise a two-component bonding material which is supplied from two different reservoirs and which is mixed when introduced into the formed channel upon retraction of the Supply tube. Again, alternatively, it is possible that the bonding material is an extruded solid material which comprises an adhesive or frictional outer surface, engaging with the side walls of the formed channel. Furthermore, photo-activated materials in which polymerization is initiated by light (such as in the UV wavelength band) may be preferred. The light source may be an optical fibre, which can be integrated in the applicator head.

Although the invention is particularly suitable for tissue bonding, the invention is not limited thereto and may be used to bond other types of materials which can be penetrated by a hollow delivery tube using a bonding material which is particularly suitable for the specific materials. Other non-limiting examples of areas in which the invention could be useful are the upholstering or clothing industry wherein textile layers may be bonded according to the present invention, in the manufacturing of filters for connecting different layers of non-woven filter material, or for instance the packaging industry in which the packaging material may be wrapped around products and may be connected using the method according to the present invention.

A device for carrying out the method according to the present invention comprises
- an outer frame,
- a hollow delivery tube, movable relative to the frame,
- an actuator connected to the delivery tube for extending and retracting the delivery tube,
- a bonding material reservoir in fluid communication with the delivery tube and
- a supply means for supplying bonding material from the reservoir to the tube during at least a part of the retracting movement of the delivery tube.

The actuator may be formed by an electrical motor such as a stepping motor, or may be formed by a hydraulic or pneumatic actuator. The supply means can be any pump mechanism suitable for supplying the bonding material such as for instance a peristaltic pump, a syringe pump, a gear pump and the like.

The reservoir for the bonding material is preferably connected to the delivery tube via a flexible duct which can accommodate a varying distance between the tube and the reservoir. The outer frame or casing may comprise a control member such as a push button which is connected to an electronic control which controls the actuator for reciprocating the delivery tube and which actuates the supply means. The device according to the present invention, which can be in the form of a pencil-shaped rod, can be simply positioned against the bonding areas whereafter the control member may be operated. Thereby a single reciprocating movement of the delivery tube follows for forming a single bond. Thereafter the device may be positioned at a new bond area and the action may be repeated. Multiple reciprocating movements of the actuator upon operating the control member are desirable to quickly form multiple bonds by moving the device parallel to the interface of the layers to be bonded. The bonding speed may be further increased by use of a tube carrier part connected to the actuator carrying at least two delivery tubes each connected to the reservoir. The tubes may be arranged in a grid pattern of for instance 10 by 10 tubes on a surface area of 1 cm². Preferably the delivery tube comprises a thin needle which can easily penetrate tissue layers to be bonded.

The bonding device according to the invention will preferably have an adjustable needle injection depth which can be adjusted in accordance with the thickness and the nature of materials that are bonded, a relatively shallow injection depth being required for instance for the bonding of blood vessels. For tissue bonding, rapid needle injection is desired to penetrate the tissue layers, which tend to be flexible and mobile. Hereby it is prevented that the tissue layers during injection are pushed forward by the delivery tube. Rapid injection is followed by a relatively slow retraction. It may also be desirable to adjust the retraction speed, possibly in combination with an adjustable glue extrusion rate, to control the overall amount of glue that is locally extruded in each formed channel. In one bonding cycle the retraction speed of the delivery tube may be constant along the retraction trajectory or may vary along this trajectory in order to form local bond parts with an increased diameter, such as a "pop rivet" shaped bond.

### Brief description of the drawings

An embodiment of the method and the device according to the present invention that is particularly suitable for tissue bonding will, by way of non-limiting example, be described with reference to the accompanying drawing. In the drawing:
Figure 1 shows a schematic side view of positioning the applicator head comprising a hollow needle on the bonding site,
Figure 2 shows a schematic side view of 5 subsequent stages of insertion of the needle beyond the interface of two apposed layers, retraction and simultaneously filling the formed channel with a bonding material,
Figures 3a and 3b show multiple parallel bond sites without and with an interface bonding layer, respectively,
Figure 4 shows a bond configuration using channels at opposite angles with respect to the interface,
Figure 5 shows a bond in the form of a pop rivet,
Figures 6 and 7 show the forming of a curved bond channel using a superelastic needle, and
Figure 8 shows a schematic side view of a device for carrying out the method according to the present invention.

### Detailed description of the drawings

### Operational principles

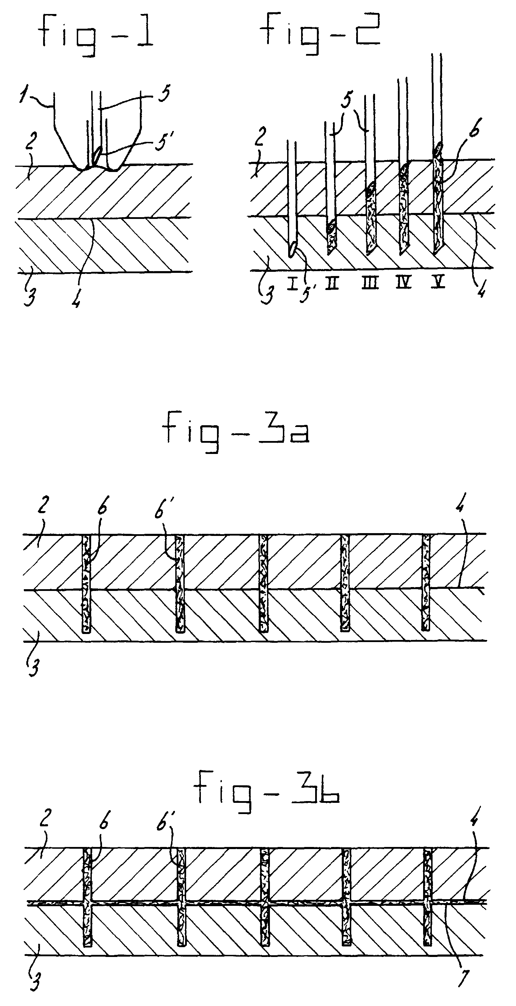

Figure 1 shows the applicator head 1 of a suturing device according to the present invention which is placed against two apposed tissue layers 2,3. The layers 2,3 are prior to bonding apposed along an interface 4. The layers 2,3 are maintaining in their properly aligned positions by the pressure exerted by the applicator head 1. In figure 1, the hollow delivery tube, which in this case is formed by a thin and pointed needle 5, is in its retracted position within the suturing device applicator head 1. The needle has an outlet opening 5'.

Figure 2 shows five subsequent stages of bonding tissue layers 2 and 3 by first injecting needle 5 rapidly in the downwards direction into the lower tissue layer 3, beyond the interface 4 in stage I. Stages II-V show that upon retraction of the needle 5, the channel formed in the tissue layers 2 and 3 is filled with a bonding material, such as an adhesive material which is supplied through the hollow needle 5. In the embodiment shown in figure 2, the adhesive material forms a glue wire 6 that completely fills the channel formed by the needle 5 and extends up to the upper surface of tissue layer 2. It is however also possible to only partly fill the channel with adhesive, such that the glue wire 6 does not extend to the outer surface of tissue layer 2.

### Tissue glue

The thickness of the glue wire 6 that is formed is determined by the diameter of the needle 5, but also by the glue extrusion volume per unit time and the needle retraction speed. Preferably the glue wire 6 is flexible and somewhat elastic. Suitable adhesives for forming the glue wire 6 are those that provide bonding between adhesive and tissue within a time interval which is acceptable to the surgeon for the particular purpose to which it is applied. This time interval will be in the order of a few seconds and less. Suitable adhesives may be activated by water such as cyanoacrylates which elicit acceptable tissue reactions and for which both curing time and viscosity can be modified to comply with the boundary conditions of the method.

In order to prevent clogging of the hollow needle 5, photo-activated tissue glues seem attractive for use in the present invention from this respect. Light may be delivered to the adhesive via an optical fibre, which may be integrated within the applicator head 1. It is also possible to use a two-component tissue adhesive wherein the mixing of the principal component and the activator can be localised at the exit of the needle 5 by the use of a double lumen needle.

### Alternative bonding configurations

Figure 3a shows a number of parallel glue wires 6,6' for bonding tissue layers 2 and 3. No adhesive is applied in the capillary fissure along the interface 4 when a sufficient bond strength can be derived from the glue wires 6,6'. In combination with the individual bond strength of each glue wire, the distance between adjacent glue wires 6,6' will determine the bond strength between tissue layers 2 and 3. As is shown in figure 3b, an additional layer of adhesive 7 can be applied in the capillary fissure along the interface 4 for increased bond strength.

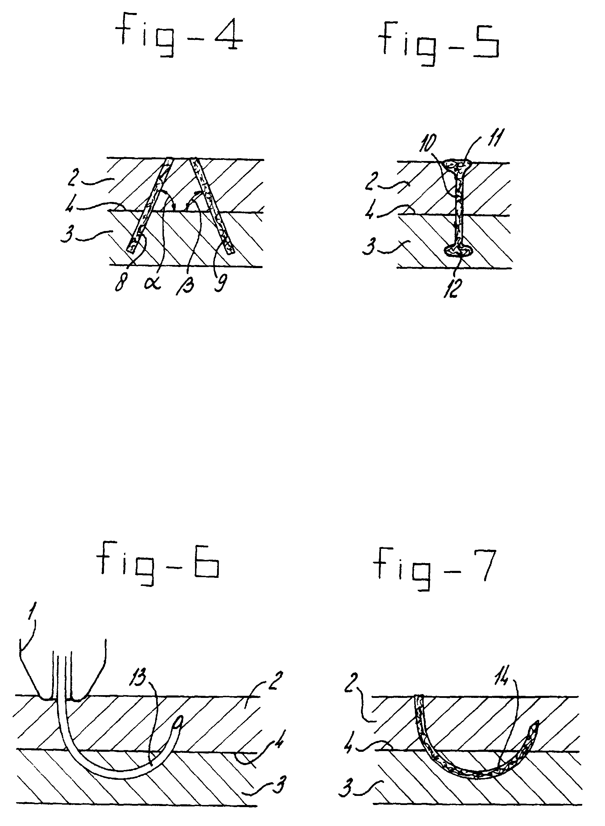

In the embodiment shown in figure 4, two glue wires 8 and 9 have been formed, either consecutively or simultaneously, having oppositely directed included angles α and β with the interface 4. In the embodiment shown in figure 5, a glue wire 10 is formed which has upper and lower parts 11,12 with an increased dimension such that a "pop rivet" shaped glue wire is formed. The parts 11,12 with increased dimension can be formed by decreasing the speed of retraction of the needle 5 at the lower and upper parts of its trajectory at a constant adhesive supply rate. Alternatively, the supply rate can be increased at the lower and upper parts of the trajectory of the needle at a constant needle retraction speed. It is also possible to vary the needle retraction speed and the adhesive supply rate simultaneously.

In the embodiment of figure 6 a superelastic needle 13 is ejected from the applicator head 1. The preformed superelastic needle 13 results in a curved trajectory through the tissue layers 3,4 by which a curved glue wire 14 is formed, as shown in figure 7.

### The bonding device

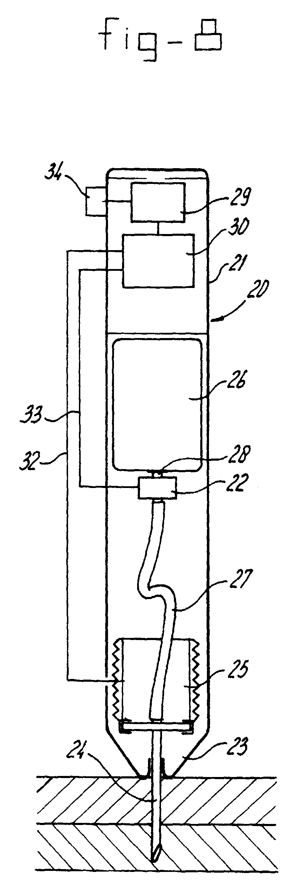

Figure 8 shows a pencil-shaped bonding device 20 according to the present invention comprising an outer frame, or casing, 21 and an applicator head 23. The applicator head 23 comprises a reciprocating hollow needle 24 which is connected to an actuator 25 for extending and retracting the needle 24 into the applicator head 23. The upper part of the needle 24 is connected to an adhesive reservoir 26 via a flexible supply duct 27. At the exit opening 28 of the glue reservoir 26, a supply means 22, such as a pump, is interposed between the flexible duct 27 and the glue reservoir 26.

At the upper end of the casing 21 a control unit 29 and a power source 30 are provided. Via electrical connections 32,33 the power source 30 is connected to the pump 22 and the actuator 25. For reasons of clarity the electrical connections 32,33 have been indicated outside the casing 21, but will in actual practise be incorporated within the casing. The control unit 29 comprises a control member 34 such as a push button which, when operated by a user, causes the control unit 29 to activate the pump 22 and the actuator 25. Furthermore, a dial (not shown in the drawing) may be provided on the casing 21 to vary the cycle of the actuator 25 in order to adjust the retraction speed of the needle 24, and to adjust the volume of adhesive supplied by the pump 22 during retraction of the needle 24. With the external dial the user may also set that upon operating the control button 34, either one cycle of the needle 24 or multiple cycles are executed.

The actuator 25 may comprise an electrical stepping motor but can also be executed as a pneumatic or hydraulic actuator. The supply duct 27 can be made of a flexible material such as to accommodate the varying distance between the needle 24 and the pump 22, wherein its length is larger than the maximum distance between the pump 22 and the upper part of needle 24 such that it can flex and bend. The duct 27 can also be made of an elastic material.

The pump 22 may be formed by a peristaltic pump operating upon the flexible duct 27, or can be formed by a rotary gear pump and the like for supplying defined amounts of adhesive from the glue reservoir 26. Instead of using a pump 22 at the exit opening 28 of the glue reservoir 26, it is also possible to execute the reservoir 26 as a bag of flexible material, wherein the supply means 22 is formed by a squeezing member contacting the outer surface of the flexible reservoir 26. In a further embodiment the reservoir 26 may be formed by a chamber within the casing 21 in which a piston is slidably mounted such that a fixed volume of adhesive is extruded from the reservoir by a defined stroke of the piston.

The power source 30 may be a mains connection, or can be formed by disposable or rechargeable batteries.

In the embodiment shown in figure 8, only a single needle 24 is used. It is however possible to connect multiple needles 24 to the actuator 25 and arrange the needles for instance in a grid pattern. Such a grid arrangement of the needles may be connected to flexible supply duct 27 via a manifold.

When a two-component glue is used, the needle 24 may be formed by a double lumen needle. Separate flexible ducts 27 will in that case be connected to the inner and outer ducts respectively. In case the glue that is used is of the photo-activated type, it is also possible to include a light source, including one or more optical fibres, in the applicator head 23 of the suturing device 20, connected to the power supply 30. For forming glue wires at an angle with the interface, such as shown in figure 4, it is possible to pivotably attach the applicator head 23 to the casing 21 such that the angle of the needle 24 can be adjusted by the user to the most convenient position with respect to outer casing 21.

## Claims

1. Method of interconnecting two objects (2,3), comprising the steps of:
- placing the objects (2,3) in a contacting relationship along an interface (4),
- penetrating the objects (2,3) with a hollow delivery tube (5) having an outlet opening (5') and forming a channel extending on each side of the interface (4), transversely hereto, and
- retracting the delivery tube (5) from the channel while supplying a bonding material via the outlet opening (5') such that the channel on both sides of the interface (4) is at least partly filled with the bonding material.

2. Method according to claim 1, wherein the bonding material is an adhesive bonding material.

3. Method according to claims 1 or 2, wherein the bonding material is a curable flowable material.

4. Method according to claims 1, 2 or 3 wherein multiple spaced-apart channels (6,6') are formed along the interface (4).

5. Method according to any of the previous claims, wherein at least two channels (8,9) are formed including opposing angles (α,β) with the interface (4).

6. Method according to any of the previous claims, wherein the retraction speed of the delivery tube near the ends of the channel is varied to form bonding material areas (11,12) of larger diameter than the mean channel diameter.

7. Method according to any of the previous claims, wherein the objects are formed by tissue.

8. Method according to any of the previous claims, wherein the penetration speed of the delivery tube (5) is relatively large compared to the retraction speed of said tube.

9. Method according to any of the previous claims, wherein the bonding material extrusion rate is varied along the retraction trajectory of the delivery tube (5).

10. Device (20) for carrying out the method according to any of the previous claims, comprising:
- an outer frame (21),
- a hollow delivery tube (24), movable relative to the frame (21),
- an actuator (25) connected to the delivery tube (24) for extending and retracting the delivery tube,
- a bonding material reservoir (26) in fluid communication with the delivery tube (24) and
- a supply means (22) for supplying bonding material from the reservoir (26) to the delivery tube (24) during at least a part of the retracting movement of the delivery tube (24).

11. Device according to claim 10, wherein the outer frame (21) comprises a control member (34) connected to the supply means (22) and to the actuator (25) for reciprocating the delivery tube (24) and for actuating the supply means (22) during one or more cycles, when the control member (34) is operated by a user.

12. Device according to claims 10 or 11, comprising a flexible and/or elastic duct (27) extending between the delivery tube (24) and the reservoir (26).

13. Device according to claims 10, 11, or 12 comprising a tube carrier part connected to the actuator (25), carrying at least two delivery tubes, each connected to the reservoir (26).

14. Device according to claim 13, wherein the delivery tubes are arranged in a grid pattern.

15. Device according to any of claims 10 to 14, wherein the delivery tube (24) comprises a needle.

16. Device according to any of claims 10 to 15, wherein the delivery tube is curved and superelastic.

17. Device according to any of claims 10 to 16, wherein the penetration speed of the actuator (25) is relatively large compared to the retraction speed thereof.

18. Device according to any of claims 10 to 17, wherein the retraction speed of the actuator (25) varies along the retraction trajectory thereof.

19. Device according to any of claims 10 to 18, wherein the supply rate of the supply means (22) varies along the retraction trajectory of the actuator (25).
